# EUROPEAN PATENT APPLICATION

(11) **EP 0 985 392 A1**
(43) Date of publication of application: **15.03.2000**
(21) Application number: 98116892.5
(22) Date of filing: 07.09.1998
(51) Int. Cl.: A61F 13/15

(54) **Absorbent article having super hydrophobic layers**

(71) Applicant: THE PROCTER & GAMBLE COMPANY, Cincinnati, Ohio 45202 (US)
(72) Inventor: Corzani, Italo, 66100 Chieti (IT); Palumbo, Gianfranco, 61348 Bad Homburg (DE)
(74) Representative: Hirsch, Uwe Thomas M.H.

(57) **Abstract**

The present invention relates to absorbent articles, for example sanitary napkins, panty liners, disposable diapers or adult incontinence products which have at least one surface of one layer with super hydrophobicity.
In particular the absorbent article has at least one layer or substrate coated with a continuos, fluorocarbon thin coating characterized by static water contact angle (WCA) higher than about 120°, preferably higher than 130°, more preferably higher than 150°.

## Description

### Field of the invention

The present invention relates to absorbent articles such as under arm sweat pads, bandages, sanitary napkins, panty liners, disposable diapers or adult incontinence products which have at least one surface of one layer provided with super hydrophobicity.

### Background of the invention

Absorbent articles are known and they are made from several layers which are joined to each other. Generally an absorbent article comprises three main elements, namely a topsheet for liquid passage, a backsheet forming the outer surface, and an absorbent core interposed between the topsheet and the backsheet.

For example, in the case of a sanitary napkin, the absorbent product comprises typically three main components: a liquid pervious topsheet, a backsheet and an absorbent core. The absorbent core is enclosed by the backsheet and the topsheet and the product is worn such that the exposed surface of the topsheet faces the wearer of the absorbent product while the exposed surface of the backsheet faces the undergarment to which the product is joint by a panty-fastening attachment means. Typically this is an adhesive but could also be a mechanical attachment.

For the purpose of improving the dryness, or the wet trough of the topsheet, or for improving the breathability of the backsheet the prior art teaches the use of hydrophobic modification of some of the above mentioned layers or the use of hydrophobic layers per se. The objective of the present invention is to improve absorbent articles such as underarm sweat pads, bandages, sanitary napkins, disposable diapers or adult incontinence products by providing at least one super hydrophobic layer according to the present invention. A known way of providing hydrophobicity is coating and particularly fluorcarbon coating.

Plasma-deposited fluorocarbon coatings are often cited in the literature as "teflon-like coatings" because their CFx (0 < x ≤ 2) composition and surface energy can be made very close to that of polytetrafluoroethylene (PTFE,-(CF₂-CF₂-)ₙ), known on the market as Teflon®.

Plasma coating processes of metals, polymers, and other substrates, with fluorocarbon films are known in the art. As an example, it is known from USP 4 869 922 and from other sources, that deposition from continuous (i.e. non modulated) radiofrequency (RF) glow discharges fed with fluorocarbons provides films, layers, tapes, plates, and differently shaped articles made of plastics, metals or other materials, with a thin fluorocarbon coating, with no other material interposed between the coating itself and the substrate. Such coatings are claimed to have very good adherence to the items processed, to be void-free, to be not porous, and to show controlled wettability characteristics, which depend on their surface chemical composition. The non modulated, continuous plasma process of the above mentioned patent leads to coatings characterized by static water contact angle (WCA) values lower than 120°.

Glow discharges treatments are also considered in US-A-5 462 781 for improving the bondability of an implantable polymer medical device or for changing the wettability of a polymer fabric. Several of the references discussed in this patent confirm non modulated, continuous plasma treatments as a means for varying the hydrophilicity or hydrophobicity of a surface.

US-A-5 034 265 discloses a non modulated, continuous plasma treatment for improving the biocompatibility of vascular grafts with a CFₓ fluorocarbon coating deposited at the inside wall of the grafts in a proper plasma reactor fed with tetrafluoroethylene (C₂F₄, TFE) at 0.2 Torr. In the preferred embodiment of the invention no other materials are interposed between the substrate and the coating.

### Summary of the invention

The present invention relates to absorbent articles such as a sanitary napkin, panty liners,disposable diaper, incontinent product, under arm sweat pads, bandages etc. having at least one surface of a layer (or substrate) treated to have super hydrophobicity. In particular the present invention relates to absorbent articles having at least one super hydrophobic surface of one layer (or substrate) which is coated by means of modulated plasma deposition of fluorocarbons.

Specifically, the present invention, having the features mentioned in the annexed claims, relates to absorbent articles having at least one layer or substrate coated with a thin, well adherent, nonporous, fluorocarbon coating with super hydrophobic properties, i.e. characterized by static water contact angle (WCA) values, measured on plane surface, higher than about 120°, preferably higher than 130°, more preferably higher than 150°. For example, absorbent articles having water vapour permeable, e.g. apertured, film backsheets treated with this method have their hydrophobicity markedly improved, e.g. can be made effectively waterproof while maintaining their previous characteristics such as permeability to gases and vapors. Consequently these breathable products show improved resistance to the wet trough of body fluids.

The present invention deals with absorbent articles having at least a treated polymeric layer or substrate whose surface is made super hydrophobic, i.e. characterized by static water contact angle (WCA) values higher than about 120°, preferably higher than 130°, more preferably higher than 150°. The substrates are preferably subjected to a modulated glow discharge plasma treatment performed with a fluorocarbon gas or vapor compound fed in a properly configured reactor vessel where the substrates are positioned. The plasma process deposits a continuous, fluorocarbon thin film with super hydrophobic surface characteristics tightly bound to the substrate.

The substrates of interest for the present invention may include a wide range of materials in form of webs, tapes, films, powders, granules, particles, woven and non-woven layers; substrates can be porous or non-porous, molded or shaped, rigid or flexible, made of polymers, textiles, papers, cellulose and its derivatives biodegradable materials, i.e. any conventional layer or substrate used in making absorbent products.

When organic synthetic resins are chosen, such substrate materials could be fabricated from polyethylene, polyacrylics, polypropylene, polyvinyl chloride, polyamides, polystyrene, polyurethanes, polyfluorocarbons, polyesters, silicone rubber, hydrocarbon rubbers, polycarbonates and other synthetic polymers. A particularly preferred polymeric substrate is polyethylene or polypropylene as used e.g. in the manufacture of sanitary products, especially of the "breathable" type as known in the art.

"Plasma," as used herein, is used in the sense of "low-temperature plasma" or "cold plasma" produced by igniting a glow discharge in a low pressure gas through a power supply. Glow discharges contain a variety of species chemically active and energetic enough to cause chemical reactions with surfaces exposed, i.e. covalent bonding to a suitable substrate material. Cold plasmas, or glow discharges, are generally produced with high frequency (from KHz to MHz and GHz) power supply (HF plasmas). Electrons, positive and negative ions, atoms, excited molecules, free radicals, and photons of various energies are formed in a cold plasma.

"Modulated plasma" means a non continuos plasma, HF plasma, i.e. a glow discharge whose driving power is pulsed between a maximum value and zero (ON/OFF pulse) or a fraction of it, at a certain frequency, with a proper pulse generator connected to the main power supply. In the case of ON/OFF pulsed systems, the time ON and time OFF values are among the experimental parameters of the process. Superimposing a triggering ON/OFF pulse to the main high frequency field which generally drives a glow discharge, alternates short continuous discharges with plasma OFF time intervals where active species still exists in the gas phase, but the effects of ions and electrons are strongly reduced. This alternating exposure to two different processes leads to unique surface modifications of substrates, which can be very different from those of continuous plasma process, as it will be shown.

"Plasma deposition" or "plasma polymerization" is the plasma process that leads to the formation of thin (0.01 - 2 µm), partly crosslinked, void-free, continuous coatings well adherent to substrates. The molecules of the gas phase are fragmented by energetic electrons, which are able to break chemical bonds; this process leads to radicals and other chemical species which are able to deposit at surfaces inside the vacuum chamber and form a thin, uniform film. The action of the plasma may also affect the surface of a polymer substrate in the early deposition time; energetic species may break bonds in the substrate with possible evolution of gas products, such as hydrogen, and formation of free radical sites which contribute to form covalent bonds between the growing film and the substrate.

It has been found that it is possible to deposit thin fluorocarbon films with super hydrophobic characteristics, i.e. showing a surprisingly high static water contact angle (WCA) value, even up to about 165°. The present invention thus refers to absorbent articles having at least one substrate of the type mentioned above with fluorocarbon films characterized by a WCA value higher than 120°, preferably higher than 130°, more preferably higher than 150°.

In particular fluorocarbon coatings with F/C ratio from about 1.50 to about 2.00 deposited on different substrates and characterized by WCA values higher than about 120°, such as between about 155° and about 165°, find useful application in absorbent articles, especially if the coatings have been deposited at the surface of different polymer substrates such as polyethylene (PE), polypropylene (PP) polyethyleneterephtalate (PET), and paper in form of films and/ or nonwovens. It should be noted that the F/C ratio could be theoretically up to 3, if the coating would be formed only by a mono-molecular layer of CF₃ groups. But the formation of intermolecular cross-links lowers the above theoretical value so that the obtained coatings, notwithstanding the fact that they contain many CF₃ groups, have a general F/C ratio in the range of about 1.50 to about 2.00.

The thickness of the coatings depends on the duration of the plasma process at different conditions, and can be kept between 0.01 and 2 µm. It has been found that the nature of the substrate materials does not influence the chemical composition or the thickness of the coatings. Coatings with WCA values up to about 165° (e.g. 165° ± 5°) were obtained.

Substrates to be treated are subjected to modulated plasma gas discharge in the presence of at least one fluorocarbon gas or vapor. Specifically, fluorocarbon gases or vapors such as tetrafluoroethylene (TFE,C₂F₄,) hexafluoropropene (HFP,C₃F₆), perfluoro-(2-trifluoromethyl-)pentene, perfluoro-(2-methylpent-2-ene) or its trimer may be used, TFE being the presently preferred choice. The plasma deposition process is preferably performed by positioning the substrate in a properly arranged plasma reactor, connecting the reactor to a source of a fluorocarbon gas or vapor, regulating flow and pressure of the gas inside the reactor, and sustaining a glow discharge in the reactor with a high frequency electric field in a pulsed (modulated) mode by means of a suitable pulsed power supply. The parameters which define the glow discharge treatment includes the feed gas or vapor, its flow rate, its pressure, the position of the substrate inside the reactor, the design of the reactor, the exciting frequency of the power supply, the input power, the time ON and the time OFF of the pulsing system. Substrates may be positioned in the "glow" region of the discharge, i.e. directly exposed to the plasma, or in the "afterglow" region, i.e. downstream in respect to the visible glow. The two positions generally result in coatings with different composition and properties; treating the substrates with modulated glow discharge results also in different coatings respect to continuous treatments.

The invention will now be described, purely by way of example, with reference to the enclosed figures of drawing, wherein:
- Figure 1 compares a conventional "continuous" RF glow discharge with an ON/OFF "modulated" RF glow discharge;
- Figure 2 portrays a typical scheme of a plasma reactor adapted for use within the context of the invention;
- Figure 3 shows a C1s ESCA signal of an uncoated polyethylene substrate wherein the signal is due only to C-H, C-C bonds of the substrate;
- Figure 4 shows a C1s ESCA signal of a PE substrate coated with a fluorocarbon coating deposited as described in example 1 (glow position, continuous mode), with WCA of 100 ± 5°; the signal is composed by components due to CF3, CF2, CF and CCF bonds of the fluorocarbon coating, and to C-H, C-C bonds due to surface contamination;
- Figure 5 shows a C1s ESCA signal of a PE substrate coated with a fluorocarbon coating deposited as described in example 1 (afterglow position, continuous mode), with WCA of 120 ± 5°; the signal is composed by components due to CF3, CF2, CF and CCF bonds of the fluorocarbon coating, and to C-H, C-C bonds due to surface contamination; and
- Figure 6 shows a C1 s ESCA signal of a PE substrate coated with a fluorocarbon coating deposited as described in example 1 (glow position, modulated mode), with WCA of 165 ± 5°; the signal is composed by components due to CF3, CF2, CF and CCF bonds of the fluorocarbon coating, and to C-H, C-C bonds due to surface contamination.

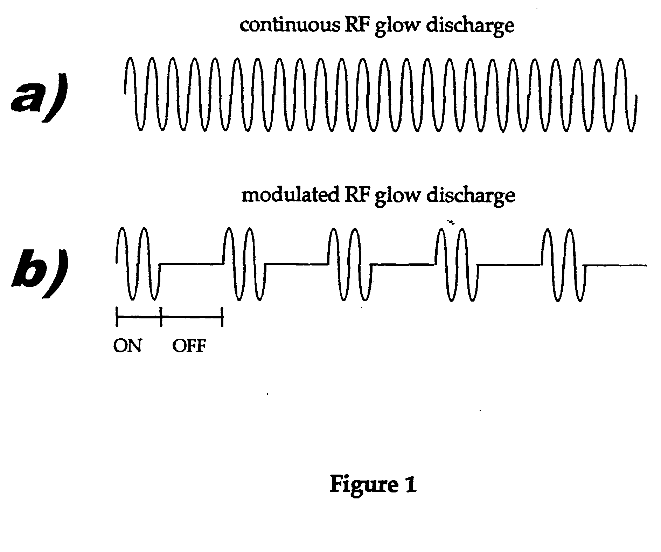

Figure 1 compares a conventional "continuous" plasma (figure 1a) with the modulated process of the invention, (figure 1b) showing pulsed alternating plasma ON with plasma OFF (i.e. no plasma) times. The two processes are schematized by referring to their driving signals.

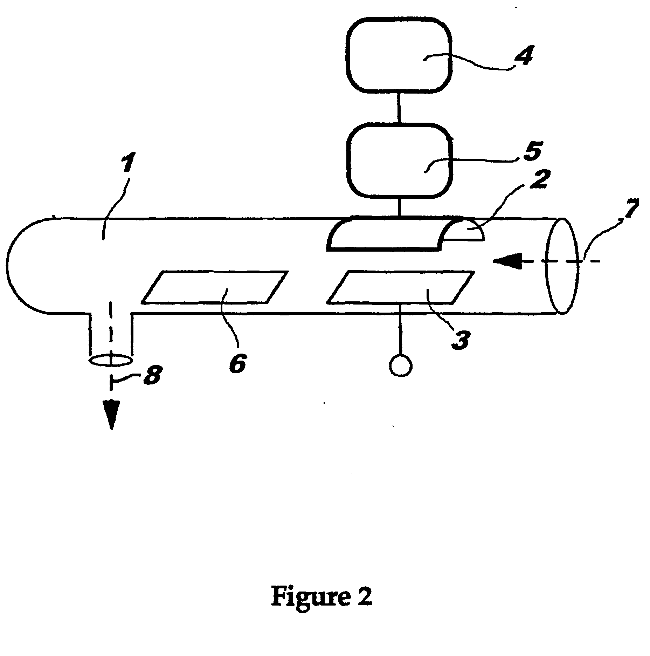

The reactor 1 schematically shown in figure 2 was utilized not exclusively for developing the deposition method object of the present invention. The reactor vacuum chamber 1 is made of Pyrex glass, is provided with an external RF powered electrode 2 and an internal grounded electrode 3. The external electrode is connected to a power supply 4 (typically a radiofrequency generator operating at e.g. 13.56 MHz) through a matching network and an ON/OFF pulse generator 5. The substrates can be treated in the "glow" region of the reactor, onto the grounded electrode 3, as well as in its "afterglow" position i.e. at an afterglow substrate holder 6. The gas/vapor is fed through a proper mass flowmeter through a gas/vapor feeding manifold 7, and its pressure, measured at the pump out exit 8 of the reactor, kept at a certain constant value with a manual valve on the vacuum connection between the reactor and its pumping unit. Even though the arrangement shown in the drawing represents a presently preferred choice, those skilled in the art will immediately recognize that pulsed energization of the plasma reactor can be achieved by different means such as direct energization by means of pulsed RF generators commonly used in radar and telecommunication techniques.

Preferably, the deposition process is performed with an RF (13.56 MHz) generator. The RF power delivered to the external electrode of the reactor is kept in the 1-500 Watts range for a power density of 0.02-10 Watt/cm². The reactor is fed with a fluorocarbon compound at a 1-100 sccm flow rate and is kept at a constant pressure of 50-1000 mTorr during the process. Preferably, the glow discharges are modulated through the pulse generator, preferably at 1-500 ms time ON and 1-1000 ms time OFF values, with respective values of about 10 ms and about 190 ms being the most preferred choice at present. The deposition process may range from a few seconds to many hours; during this time a uniform fluorocarbon coating is deposited on the substrates positioned in the glow as well as on those in the afterglow region. The deposition rate, a typical one being in the 20 - 400 Å/min range, was measured by weighing (weight/time) the substrates before and after the discharge, or by measuring the thickness of the coatings (thickness/time) with an Alpha Step profilometer. The deposition rate and the chemical composition of the coating depend on the experimental conditions (pressure, power, substrate position time ON, time OFF, gas feed and flow rate) of the discharge.

The coatings obtained are uniform over the entire surface of the substrate; when deposited on flat, smooth substrates, their hydrophobic character has been estimated through their static WCA value, as measured with a WCA goniometer. WCA values in the range about 120° to about 165°, corresponding to a critical surface tension lower than that of PTFE (18 dynes/cm) have been measured for fluorocarbon CFx coatings, when x ranges between about 1.50 and about 2.00. The chemical composition of coatings is preferably determined by Electron Spectroscopy for Chemical Analysis (ESCA) within the sampling depth of the technique (about 100 Å). The adherence of the coating to the substrate is very good.

The following examples are given for the purpose of still better illustrating the inventive concept of the present invention, and for highlighting the advantages of using modulated over continuous treatments.

### EXAMPLE 1

Three sets of substrates of silicon, PE and PP, of areas in the range of 2-10 cm² per substrate, were positioned onto the grounded electrode 3 of the reactor schematized in Figure 2. A similar set of substrates was positioned in the afterglow position at 6. C₂F₄ was set to feed continuously the reactor at 6 sccm, and the pressure set at 300 mTorr. The RF generator was connected to the reactor and allowed to sustain the discharge with 50 Watt of input power for 90 min, then switched off.

Another glow discharge was subsequently run with a similar set of substrates positioned in the glow position and no substrates in the afterglow position, under the same conditions described above except for the fact that modulation was effected at 10 ms time ON and 190 ms time OFF through the pulse generator.

At the end of the two discharges the substrates were extracted from the reactor and their WCA measured. The WCA values shown in Table 1 were found, which are compared to the WCA values of the unprocessed substrates. A deposition rate of 30 ± 5 Å/min was measured for the coatings deposited in the modulated mode.

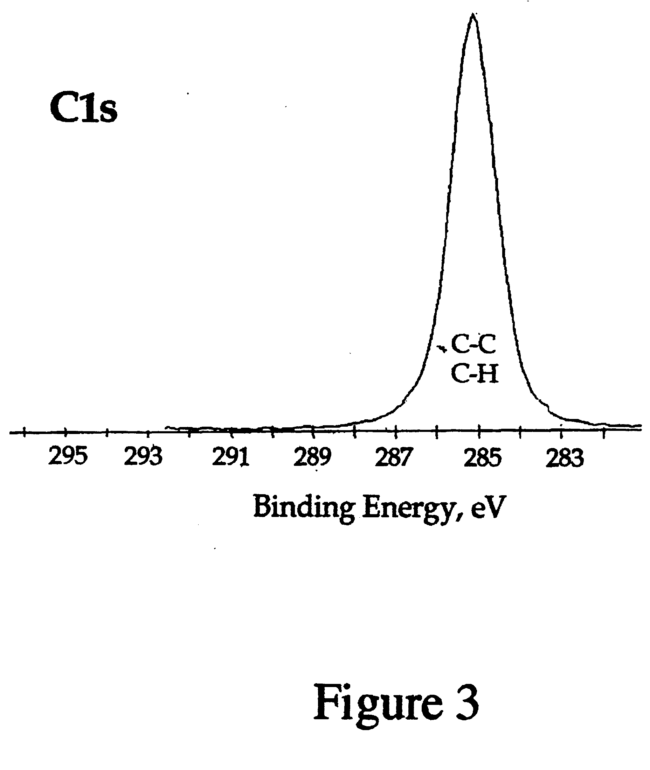

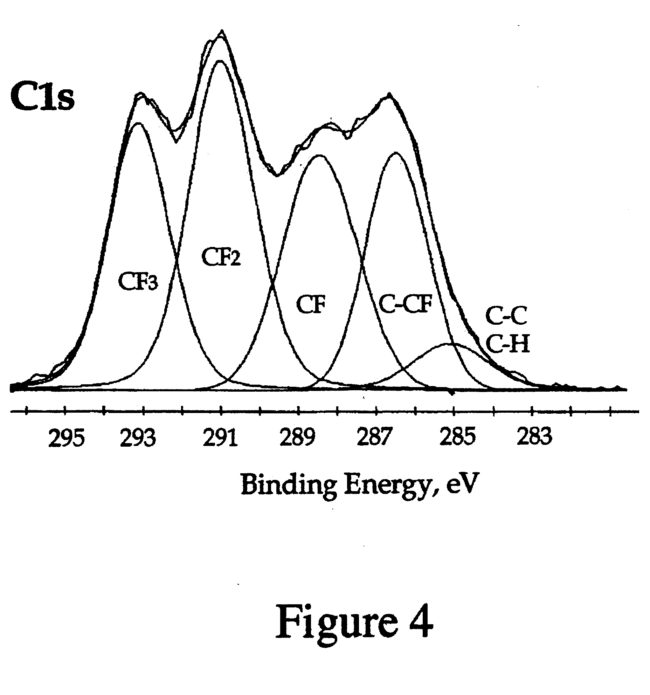

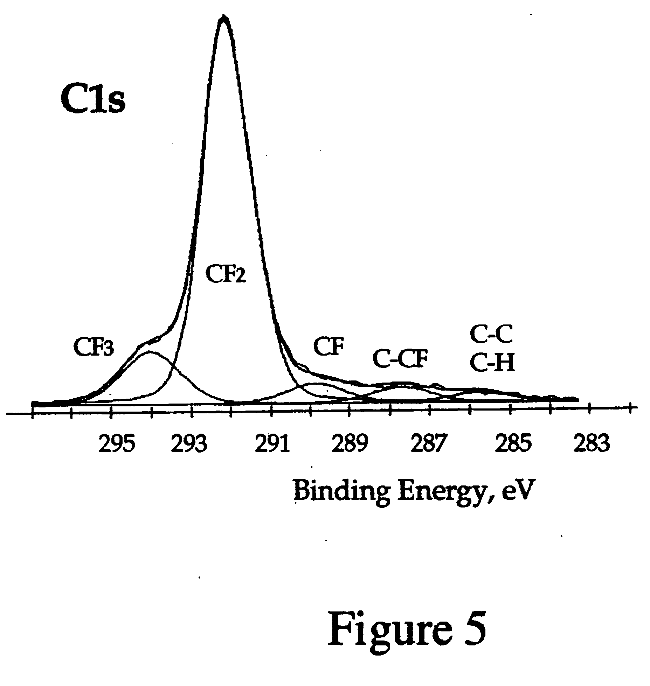

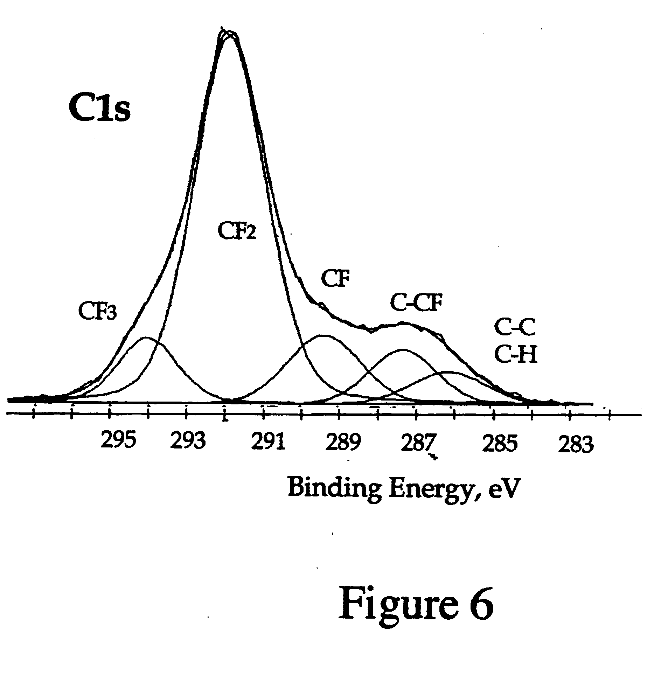

Other substrates, treated in the two modes, where analysed with the ESCA technique. Their surface composition resulted to be entirely composed by carbon and fluoride, according to the results shown in Tables 2a-c. No other elements were detected (e.g. Si for silicon substrates), which means that the coatings are continuous. The C1s spectrum of the uncoated PE substrate is shown in Figure 3, while the C1s spectra of PE samples coated as described above are shown in Figures 4, 5 and 6, respectively.

**Table 1**

| SUBSTRATE | Si | PE | PP |
|---|---|---|---|
| WCA unprocessed | 15°± 3° | 95°± 3° | 85°± 3° |
| WCA continuous discharge (glow position) | 100°± 5° | 100° ± 5° | 100° ± 5° |
| WCA continuous discharge (afterglow position) | 120° ± 5° | 120° ± 5° | 120° ± 5° |
| WCA modulated discharge (glow position) | 165° ± 5° | 165° ± 5° | 165° ± 5° |

**Table 2a**

| ESCA results for the continuous discharge (glow position) of Example 1 | | | |
|---|---|---|---|
| COATED SUBSTRATE | Si | PE | PP |
| carbon atomic % | 43.3 | 42.4 | 42.9 |
| fluorine atomic % | 56.7 | 57.6 | 57.1 |
| F/C ratio | 1.31 | 1.36 | 1.33 |

**Table 2b**

| ESCA results for the continuous discharge (afterglow position) of Example 1 | | | |
|---|---|---|---|
| COATED SUBSTRATE | Si | PE | PP |
| carbon atomic % | 34.4 | 33.8 | 34.1 |
| fluorine atomic % | 65.6 | 66.2 | 65.9 |
| F/C ratio | 1.91 | 1.96 | 1.93 |

**Table 2c**

| ESCA results for the modulated discharge (glow position) of Example 1 | | | |
|---|---|---|---|
| COATED SUBSTRATE | Si | PE | PP |
| carbon atomic % | 36.4 | 36.2 | 36.7 |
| fluorine atomic % | 63.6 | 63.8 | 63.3 |
| F/C ratio | 1.75 | 1.76 | 1.72 |

### EXAMPLE 2

Three sets of substrates of glass, silicon and PE, of areas in the range of 2-10 cm² per substrate, were positioned onto the grounded electrode 3 of the reactor schematized in Figure 2. A similar set of substrates was positioned in the afterglow position. C₃F₆ was set to feed continuously the reactor at 5 sccm, and the pressure set at 300 mTorr. The RF generator was connected to the reactor and allowed to sustain the discharge with 50 Watt of input power for 60 min, then switched off.

Another glow discharge was subsequently run with a similar set of substrates positioned in the glow position and no substrates in the afterglow, under the same conditions described above except for the fact that modulation was effected at 10 ms time ON and 90 ms time OFF through the pulse generator.

At the end of the two discharges the substrates were extracted from the reactor and their WCA measured. The WCA values shown in Table 3 were found, which are compared to the WCA values of the unprocessed substrates. A deposition rate of 70 ± 5 Å/min was measured for the coatings deposited in the modulated mode.

Other substrates, treated in the two modes, where analysed with the ESCA technique; their surface composition resulted to be entirely composed by carbon and fluoride, according to the results shown in Tables 4a-c. Also for this case since no other elements were detected (e.g. Si for silicon and glass substrates), the coatings were assumed to be continuous.

**Table 3**

| SUBSTRATE | glass | Si | PE |
|---|---|---|---|
| WCA unprocessed | 35° ± 3° | 15°± 3° | 95° ± 3° |
| WCA continuous discharge (glow position) | 105° ± 5° | 105° ± 5° | 105° ± 5° |
| WCA continuous discharge (afterglow position) | 120° ± 5° | 120° ± 5° | 120° ± 5° |
| WCA modulated discharge (glow position) | 120° ± 5° | 120° ± 5° | 120° ± 5° |

**Table 4a**

| ESCA results for the continuous discharge (glow position) of Example 2 | | | |
|---|---|---|---|
| COATED SUBSTRATE | glass | Si | PE |
| carbon atomic % | 42.7 | 42.1 | 41.5 |
| fluorine atomic % | 57.3 | 57.9 | 58.5 |
| F/C ratio | 1.34 | 1.37 | 1.41 |

**Table 4b**

| ESCA results for the continuous discharge (afterglow position) of Example 2 | | | |
|---|---|---|---|
| COATED SUBSTRATE | glass | Si | PE |
| carbon atomic % | 37.1 | 38.3 | 38.1 |
| fluorine atomic % | 62.9 | 61.7 | 61.9 |
| F/C ratio | 1.69 | 1.61 | 1.62 |

**Table 4c**

| ESCA results for the modulated discharge (glow position) of Example 2 | | | |
|---|---|---|---|
| COATED SUBSTRATE | glass | Si | PE |
| carbon atomic % | 38.1 | 39.9 | 39.2 |
| fluorine atomic % | 61.9 | 60.1 | 60.8 |
| F/C ratio | 1.62 | 1.51 | 1.55 |

### EXAMPLE 3

Three set of substrates of polished silicon, polyethyleneterephtalate (PET), and 3 mm thick FAM (Functional Absorbent Material), an hydrophilic absorbent material made according to the teachings of USP 5 260 345, of areas in the range of 2-10 cm² per substrate, were positioned onto the grounded electrode 3 of the reactor schematized in Figure 1. C₂F₄ was set to feed continuously the reactor at 5 sccm, and the pressure set at 400 mTorr. The RF generator was connected to the reactor and allowed to sustain the discharge for 20 min in the modulated mode (10 ms time ON; 190 ms time OFF) with 75 Watt of input power. At the end of the discharge the substrates were extracted from the reactor, and their WCA measured. The values shown in Table 5 were found, which are compared to the WCA values of the unprocessed substrates. A deposition rate of 300 ± 10 Å/min was measured.

Other substrates where ESCA analysed; their surface composition resulted to be entirely composed by carbon and fluoride, according to the results shown in Table 6. No other elements have been detected (e.g. Si for silicon substrates, and O for PET substrates), thus the coatings can be assumed to be continuous.

The coated FAM substrate was cut along its thickness, and the freshly cut surface, which was not directly exposed to the discharge) analysed by WCA and ESCA measurements. The data shown in Table 7 demonstrate that the thick FAM sample was treated not only on the surface exposed to the glow, but also inside its bulk, which demonstrates that the plasma treatment is able to penetrate through porous substrates.

**Table 5**

| SUBSTRATE | PET | Si | FAM |
|---|---|---|---|
| WCA unprocessed | 75°± 3° | 15°± 3° | WCA unmeasurable, the drop is adsorbed by the material |
| WCA modulated discharge (glow position) | 155° ± 5° | 155°±5° | 155° ± 10° |

**Table 6**

| ESCA results for the modulated discharge (glow position) of Example 3 | | | |
|---|---|---|---|
| COATED SUBSTRATE | PET | Si | FAM |
| carbon atomic % | 34.8 | 35.6 | 34.0 |
| fluorine atomic % | 65.2 | 64.4 | 66.0 |
| F/C ratio | 1.87 | 1.81 | 1.94 |

**Table 7**

| ESCA results for the treated FAM sample of Example 3 cut just after the treatment | |
|---|---|
| SUBSTRATE | bulk FAM, cut after the treatment |
| WCA(modulated discharge) | the drop of water is not absorbed |
| | the material is evidently much more hydrophobic respect to the untreated one |
| | WCA is unmesurable due to surface |
| | roughness |
| carbon atomic % | 38.5 |
| fluorine atomic % | 61.5 |
| F/C ratio | 1.60 |

For a better understanding of the present invention but without limitation the main components of an absorbent article will be now described. As stated before, typically such articles are of layered construction with each layer or group of layers and the article itself having a garment facing surface which is oriented to face in the direction of a garment during use of the article and a wearer facing surface facing in the opposite direction. Typically such articles comprise a liquid pervious topsheet layer forming the wearer facing surface of the article, an absorbent core layer and a backsheet layer forming the garment facing surface of the article. The absorbent core is interposed between the topsheet and the backsheet.

It should be appreciated that one or more of the surfaces of the preceding or following described layers can be super hydrophobic coated according to the method above , when it is useful to add hydrophobicity to one or both surfaces of one or more layers of the absorbent product for improving the performance of the absorbent product.

However it is emphasized that while the art describes the use of hydrophobic layers in the context of absorbent products, there are no discloses of absorbent articles such as sanitary napkins, disposable diapers or adult incontinence products, etc. comprising at least one super hydrophobic layer according to the present invention with the associated advantages.

### Main Absorbent Article Components

### Topsheet

The topsheet layer is compliant, soft feeling, and non-irritating to the wearer's skin. The topsheet material also can be elastically stretchable in one or two directions. Further, the topsheet is inherently fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present are selected from high loft nonwoven topsheets and apertured formed film topsheets. Apertured formed films are especially preferred for the topsheet because they are pervious to body exudates and yet non-absorbent and have a reduced tendency to allow fluids to pass back through and rewet the wearers skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. 5,006,394. Particularly preferred microapertured formed film topsheets are disclosed in U.S. patent 4,609,518 and U.S. patent 4,629,643. The preferred topsheet for the present invention is the formed film described in one or more of the above patents and marketed on sanitary napkins by The Procter & Gamble Company of Cincinnati, Ohio as "DRI-WEAVE."

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The material forming the apertures of the formed film topsheet can be hydrophilic so as to help liquid to transfer through the topsheet faster than if the material was not hydrophilic. In a preferred embodiment, surfactant is incorporated into the polymeric materials of the formed film topsheet such as is described in U.S. Patent Application Serial No. 07/794,745, filed on November 19, 1991 or EP-A-166 058. Alternatively, the body surface of the topsheet can be made hydrophilic by treating it with a surfactant such as is described in U.S. 4,950,254.

### Absorbent core

The absorbent core typically includes the following components: (a) optionally a primary fluid distribution layer; (b) optionally, but preferably, a secondary fluid distribution layer; (c) a fluid storage layer; (d) optionally a fibrous ("dusting") layer underlying the storage layer; and (e) other optional components.

### a. Primary Fluid Distribution Layer

One optional component of the absorbent cores according to the present invention is the primary fluid distribution layer. This primary distribution layer typically underlies the topsheet and is in fluid communication therewith. The topsheet transfers the acquired menstrual fluid to this primary distribution layer for ultimate distribution to the storage layer. This transfer of fluid through the primary distribution layer occurs not only in the thickness, but also along the length and width directions of the absorbent product.

### b. Optional Secondary Fluid Distribution Layer

Also optional but a preferred component of the absorbent cores according to the present invention is a secondary fluid distribution layer. This secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized.

### c. Fluid Storage Layer

Positioned in fluid communication with, and typically underlying the primary or secondary distribution layers, is a fluid storage layer comprising certain absorbent gelling materials and/or other absorbent materials, which can form the carrier matrix for the absorbent gelling materials. Absorbent gelling materials are usually referred to as "hydrogels," "superabsorbent" "hydrocolloid" materials. Absorbent gelling materials are those materials that, upon contact with aqueous fluids, especially aqueous body fluids, imbibes such fluids and thus form hydrogels. These absorbent gelling materials are typically capable of absorbing large quantities of aqueous body fluids, and are further capable of retaining such absorbed fluids under moderate pressures. These absorbent gelling materials are typically in the form of discrete, nonfibrous particles.

The fluid storage layer can comprise solely absorbent gelling materials) or these absorbent gelling materials can be dispersed homogeneously or non-homogeneously in a suitable carrier or it can comprise solely an absorbent carrier material.

Suitable carriers include cellulose fibers, in the form of fluff, tissues or paper such as is conventionally utilized in absorbent cores. Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferred synthetic fibers have a denier of from about 3 denier per filament to about 25 denier per filament, more preferably from about 5 denier per filament to about 16 denier per filament. Also preferably, the fiber surfaces are hydrophilic or are treated to be hydrophilic. The storage layer can also include filler materials, such as Perlite, diatomaceous earth, Vermiculite, etc., that lower rewet problems.

If dispersed non-homogeneously in a carrier, the storage layer can be locally homogeneous, i.e. have a distribution gradient in one or several directions within the dimensions of the storage layer. Non-homogeneous distribution can also refer to laminates of carriers enclosing absorbent gelling materials partially or fully. If laminates are used they can be formed with or without absorbent gelling particles. In particular thermally bonded air laid fibrous sheets or laminates and/or thermally bonded wet laid sheets or laminates have been found useful, especially in the context of panty liners when no absorbent gelling material is used.

Preferably, the storage layer comprises from about 15 to 100% absorbent gelling materials and from 0 to about 85% carrier. More preferably, the storage layer comprises from about 30 to 100 %, most preferably from about 60 to 100% absorbent gelling materials and from 0 to about 70 %, most preferably from 0 to about 40 %, carrier.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 and reissued as RE 32,649. Preferred monomers include acrylic acid, methacrylic acid, and 2-acrylamido-2-methyl propane sulfonic acid. Acrylic acid itself is especially preferred for preparation of the polymeric gelling material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof. Especially preferred are the polyacrylates and polyacrylate grafted starch.

While these absorbent gelling materials are typically in particle form, it is also contemplated that the absorbent gelling material can be in the form of macrostructures such as fibers, sheets or strips.

### d. Optional Fibrous ("Dusting") Layer

An optional component for inclusion in the absorbent cores according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of laminates or of macrostructures such as fibers, sheets or strips, this fibrous "dusting" layer need not be included. However, because this "dusting" layer provides some additional fluid-handling capabilities such as rapid wicking of fluid along the length of the pad, its inclusion is typically preferred in absorbent cores according to the present invention.

### e. Other Optional Components

The absorbent cores according to the present invention can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core.

Another component which can be included in the absorbent core and preferably is provided close to or as pad of the primary or secondary fluid distribution layer are odor control agents. These can be selected from active carbon or coated active carbon to conced the color, suitable zeolite or clay materials, or suitable zeolites and silicas are optionally incorporated in the absorbent core. Also absorbent gelling material in combination with certain zeolites and silicas have been found useful. These components can be incorporated in any desired form but often are included as discrete, non-fibrous particles.

### Backsheet

The backsheet layer prevents the exudates absorbed and contained in the absorbent core from wetting articles that contact the absorbent article such as pants, pyjamas and undergarments. The backsheet is preferably impervious to liquids (e.g., menses and/or urine) and is preferably manufactured from a thin plastic film, although other flexible liquid impervious materials can also be used. The backsheet needs to be compliant and will readily conform to the general shape and contours of the human body. The backsheet preferably also can have characteristics allowing it to elastically stretch in one or two directions.

The backsheet primarily prevents the exudates absorbed and contained in the absorbent core from wetting articles that contact the absorbent product such as underpants, pants, pyjamas and undergarments. The backsheet is preferably impervious to liquids (e.g. menses and/or urine) and usually manufactured from a thin plastic film.

Preferably, the backsheet also provides breathability to the absorbent article by being at least water vapour permeable, preferably air permeable. The backsheet can be a laminate material e.g. of a combination with microporous film and/or non-woven material, and/or apertured formed film. Breathability if desired can be limited to the periphery or the center of the backsheet or it can be across the whole backsheet.

The backsheet can comprise a woven or nonwoven material, polymeric films such as thermoplastic films of polyethylene or polypropylene, or composite materials such as a film-coated nonwoven material. It is also possible that the backsheet is provided by open cell foam material which are not treated to be absorbent such foams - however treated to be absorbent - are well known and are discribed as microporous polyurethan foam made from high inter phase emulsions. Conventionally in non-breathable articles the backsheet is a polyethylene film having a thickness of from about 0.012mm to about 0.051 mm.

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet can permit vapors to escape from the absorbent structure (i.e., be breathable) while still preventing exudates from passing through the backsheet. In particular for sanitary napkins it is possible to utilise apertured hydrophobic, polymeric films having directional liquid transport - such as those disclosed above for topsheets - towards the absorbent structure, as breathable backsheets. More preferably, a breathable backsheet has a two layer structure where the apertured film is lined with a non woven or a microporous film. In this case, preferably the non woven or the microporous film forms the garment facing surface of the sanitary napkin on which usually a panty fastening adhesive is placed.

### Super hydrophobicity in absorbent articles

It will be appreciated by those skilled in the art that the structural elements are disclosed for the purpose of illustration for the present invention. It is obvious that providing one or several of the surfaces of the layered elements in an absorbent article with super hydrophobicity can be exceptionally beneficial for the overall performance of the article. Which surface or surfaces should be provided with super hydrophobicity can easily be defined by those skilled in the art in dependence of desired performance improvement and with simple experimentation or trial of the article. Particularly beneficial embodiments are listed in the following examples.

Particularly beneficial for low rewet and skin dryness in absorbent articles is the incorporation of super hydrophobic coating on the wearer facing surface of the topsheet in contact with the wearer's skin. This will put a high aqueous liquid driving force into the article for any liquid deposited on that surface.

Another embodiment are breathable absorbent articles. For them the use of a super hydrophobic surface treatment on the garment facing surface of the article or on one of the garment facing surfaces of the layers between the backsheet and the storage layer of the absorbent structure is highly beneficial in preventing soiling of liquid through the breathable backsheet especially if it comprises liquid permeable apertures.

Selection which layer to coat in a breathable absorbent article depends on the expected benefit and purpose of the coating. For example coating of an apertured formed film structure may provide better soiling protection if this structure does not provide the garment facing outside surface than coating of a non-woven which provides the garment facing outside surface of the article. The reason is that the coating may provide a different macroscopical performance of liquid passage functionality for super hydrophobic coated non-wovens or wovens versus apertured films which inherently can have certain liquid passage restriction functions at least in one direction.

In general the use of coatings of super hydrophobicity should be such that the structural liquid driving force ( often also referred to as capalarity ) and the surface material related driving force (typically referred to as hydrophobicity / hydrophilicity due to material characteristics rather than structural characteristics) are balanced such that the storage layer in the absorbent core is the location within the article from which liquid has no natural tendency to escape. In physical terms the presence of liquid in the liquid storage layer of the absorbent core should be the status of highest entropy (lowest free energy) for migration of liquide.

## Claims

1. A disposable absorbent article comprising at least three layers:
• a generally liquid permeable topsheet,
• a generally liquid impermeable backsheet
• an absorbent core between said topsheet and said backsheet,
• each layer comprising two surfaces,
characterized in that at least one surface of at least one of said layers is surface treated with a coating and said treated surface has a static water contact angle (WCA) higher than about 120°, preferably higher than about 130°, most preferably between about 150° and about 165°.

2. An absorbent article according to claim 1 characterized in that said coating is a fluorocarbon coating.

3. An absorbent article according to claim 2 characterized in that said coating exhibits a fluorine/carbon ratio (F/C) of between about 1.50 and about 2.00, preferably between about 1.60 and about 1.95.

4. An absorbent article according to any of the preceding claims which article is water vapour permeable and wherein said at least one surface of said at least one layer is comprised in said backsheet.

5. An absorbent article according to claim 4 characterized in that said at least one layer is an apertured film layer and the garment facing surface of said apertured film is coated with fluorocarbons.

6. An absorbent article according to any of the preceding claims wherein said at least one surface of said at least one layer is comprised in said topsheet.

7. An absorbent article according to claim 6 characterized in that said at least one layer is an apertured film layer and the wearer facing surface of said apertured film is coated with fluorocarbons.

8. An absorbent article according to any of the preceding claims in which said article is a sanitary napkin, a panty liner, a disposable diaper, or an incontinent product.
